# EUROPÄISCHE PATENTSCHRIFT

(11) **EP 0 567 105 B1**
(45) Veröffentlichungstag und Bekanntmachung des Hinweises auf die Patenterteilung: **08.01.1997**
(21) Anmeldenummer: 93106504.9
(22) Anmeldetag: 21.04.1993
(51) Int. Cl.: A61F 13/15

(54) **Wegwerfwindeln**
Diapers
Couches à jeter

(30) Priorität: 21.04.1992 JP 101340/92
(43) Veröffentlichungstag der Anmeldung: 27.10.1993
(73) Patentinhaber: UNI-CHARM CORPORATION, Kawanoe-shi Ehime-ken (JP)
(72) Erfinder: Tanji, Hiroyuki, Kawanoe-shi, Ehime-ken (JP); Wada, Ichiro, Kawanoe-shi, Ehime-ken (JP); Ono, Yoshio, Kawanoe-shi, Ehime-ken (JP); Soga, Hiroyuki, Kawanoe-shi, Ehime-ken (JP)
(74) Vertreter: Sperling, Rüdiger, Dipl.-Ing.

(56) Entgegenhaltungen:
- EP-A- 0 309 246
- WO-A-91/08717
- WO-A-92/14429
- GB-A- 2 161 059

## Beschreibung

Diese Erfindung betrifft eine Wegwerfwindel, die zum Absorbieren und Aufnehmen von Ausscheidungen aus dem menschlichen Körper verwendet wird.

Die japanische Gebrauchsmusteranmeldung Amtsblatt-Nr. 1974-120439 zeigt eine Windeleinlage mit einer Decklage auf, die in ihrem Mittelbereich mit einer Öffnung versehen ist, die sich in Längsrichtung weiter als in Querrichtung der Decklage erstreckt, wobei die Öffnung entlang ihrem Umfangsrand mit einem in Längsrichtung dehnbaren elastischen Element versehen ist, das eine elastische Linie in Form eines geschlossenen Ringes bildet. Die japanische Patentanmeldung Amtsblatt-Nr. 1986-41304 zeigt ebenfalls eine Wegwerfwindel mit einer Decklage auf, die in ihrem Mittelbereich mit einer Öffnung versehen ist, die sich in Längsrichtung weiter als in Querrichtung der Decklage erstreckt, wobei die Öffnung entlang ihren seitlich einander gegenüber liegenden Seitenrändern mit elastischen Elementen jeweils versehen ist. Bei dieser Windeleinlage und Windel, die beide mit Öffnungen versehen sind, fließen Ausscheidungen durch diese Öffnung in zwischen der Decklage und einer getrennt vorgesehenen Decklage, die unter der zuerst erwähnten Decklage liegt, gebildete Taschen, und werden darin gehalten. Eine ähnliche Windel ist aus dem Dokument WO 91/08717 bekannt.

Sicherlich wird ein Ausscheidungsfluß zuverlässig in die Öffnung allein durch eine annähernde Dimensionierung der Öffnung, das heißt in angemessen großen Abmessungen, geführt. Eine derartige Maßnahme führt jedoch zu einem entsprechenden Anwachsen der Fläche, in der die vorstehend erwähnte getrennt vorliegende Decklage, die unterhalb der die Öffnung aufweisenden hautberührenden Decklage freiliegt, worauf in der Folge der von der hautberührenden Decklage zu bewirkende Effekt in nicht akzeptierbarer Weise verringert wird. Demgemäß muß die Öffnung hinsichtlich ihrer Abmessungen unter Berücksichtigung eines derartigen Problems konstruiert sein. Auch wenn die Öffnung in geeigneter Weise konstruiert ist, kann die Öffnung jedoch oftmals nicht mit der idealen Anordnung im Schrittbereich des Windelträgers übereinstimmend ausgerichtet sein, in Abhängigkeit von der Art und Weise, in der die Hilfsperson die Windel dem Träger angelegt hat oder der Träger bzw. die Trägerin sich mit der an seinem oder ihrem Körper angelegten Windel bewegt. In einem solchen Fall können Ausscheidungen teilweise über die Oberseite der hautberührenden Decklage fließen und sich verteilen und die Haut des Trägers bzw. der Trägerin kann mit Ausscheidungen verschmiert werden. Die Windel wie auch die Windeleinlage nach dem Stand der Technik, wie vorstehend beschrieben, können ein derartiges Problem nicht lösen und auch eine die Lösung nahelegende Beschreibung kann in den vorstehend erwähnten Veröffentlichungen nicht gefunden werden.

Es ist eine wesentliche Aufgabe der Erfindung, eine Wegwerfwindel aufzuzeigen, die dergestalt verbessert ist, daß die Öffnung in wirksamer Weise verhindert, daß Ausscheidungen über die Oberseite der hautberührenden Decklage fließen und sich verteilen, auch wenn die Ausscheidungen teilweise an der Öffnung vorbeigelangen.

### BESCHREIBUNG DER ERFINDUNG

Vorstehend ausgeführte Aufgabe wird gemäß der Erfindung durch eine Wegwerfwindel erzielt, die eine flüssigkeitsdurchlässige erste Decklage, eine flüssigkeitsundurchlässige Außenlage, einen flüssigkeitsabsorbierenden Kern, der zwischen der ersten Decklage und der Außenlage liegt, und eine flüssigkeitsbeständige zweite Decklage umfaßt, die über der ersten Decklage liegt, wobei die zweite Decklage im wesentlichen in ihrem Mittelbereich mit einer Öffnung versehen ist, die in Längsrichtung weiter als in Querrichtung der zweiten Decklage sich erstreckt, wobei die zweite Decklage entlang ihrem äußeren Umfang mit der ersten Decklage verbunden ist, und wobei in Längsrichtung dehnbare elastische Elemente entlang dem Umfangsrand der Öffnung auf der zweiten Decklage befestigt sind, dadurch gekennzeichnet, daß die Öffnung mit zwei ersten Klappen versehen ist, deren erste innere Seitenränder einander gegenüberliegend und voneinander beabstandet angeordnet sind, und zwei zweite Klappen, deren zweite Innenränder jeweils einander gegenüberliegend und voneinander beabstandet angeordnet sind, wobei der Abstand zwischen ihnen größer ist als der Abstand, in dem die ersten inneren Seitenränder voneinander beabstandet sind, wobei die beiden zweiten Klappen sich kontinuierlich an die unteren Seitenränder der jeweiligen ersten Klappen anschließen, und die zweiten inneren Seitenränder zwischen den ersten inneren Seitenrändern und den zugehörigen unteren Seitenrändern der jeweiligen ersten Klappen positioniert sind, so daß diese zweiten inneren Seitenränder sich über die oberen Oberflächen der jeweiligen ersten Klappen erheben können und wenigsten die ersten inneren Seitenränder jeweils die elastischen Elemente enthalten.

Vorzugsweise werden die ersten und zweiten Klappen durch Falten der gegenüberliegenden inneren Seitenränder der Öffnung gebildet.

Vorzugsweise umfaßt die zweite Decklage zwei Lagenelemente, die jeweils die ersten und zweiten Klappen enthalten.

Vorzugsweise sind die inneren Seitenränder der Lagenelemente, die beidseits in der Nähe der in Längsrichtung gegenüberliegenden Enden einander gegenüberliegen, miteinander verklebt, so daß diese verklebten inneren Seitenränder voneinander getrennt werden können, ohne daß an den Lagenelementen selbst Risse auftreten.

Bei der gemäß der Erfindung wie vorstehend beschrieben aufgebauten Windel erheben sich die ersten und zweiten Klappen, die die Öffnung umgeben, über die erste Decklage, wenn sich die elastischen Elemente während der tatsächlichen Benutzung der Windel zusammenziehen. In diesem Zustand der Windel fließen im wesentlichen alle Ausscheidungen durch die zwischen den ersten Innenrändern der jeweiligen ersten Klappen gebildete Öffnung in Taschen, die zwischen den ersten und zweiten Decklagen gebildet sind, wobei feste Ausscheidungen innerhalb dieser Taschen gehalten werden, während flüssige Ausscheidungen durch die erste Decklage in den Absorptionskern absorbiert werden und darin festgehalten werden. Ein Teil der Ausscheidungen, der an der Öffnung vorbeifließt, wird von den zweiten Klappen eingedämmt und dadurch kann dieser Teil der Ausscheidungen daran gehindert werden, sich ausbreitend über die obere Fläche der zweiten Decklage zu fließen.

### KURZE BESCHREIBUNG DER ZEICHNUNG

Die Erfindung wird anhand eines Beispiels unter Bezug auf die beiliegenden Figuren beschrieben, wobei
- Fig. 1: eine perspektivische Darstellung der Innenseite einer Wegwerfwindel, die als eine Ausführungsform der vorliegenden Erfindung aufgebaut ist, zeigt;
- Fig. 2: eine Schnittdarstellung dieser Ausführungsform in vergrößertem Maßstab entlang der Linie 2-2 in Fig. 1 zeigt;
- Fig. 3: eine Draufsicht zur Erläuterung der Weise, in der endlose elastische Elemente an einem endlosen Bahnelement als Ausgangsmaterial für eine zweite Decklage angebracht werden, zeigt;
- Fig. 4: eine Draufsicht zeigt, die die Weise erläutert, in der ein endloses Lagenelement mit Schneidlinien versehen wird, so daß das Lagenelement beginnend am einen Seitenrand des Lagenelementes zweimal umgefaltet werden kann, um erste und zweite Klappen zu bilden, sowie den Ausschnitt, der mit dem Gegenausschnitt zusammenwirkt, um eine Öffnung zu bilden;
- Fig. 5: eine Draufsicht zur Erläuterung der Weise zeigt, in der ein Teil des Lagenelementes unter Nutzung der Schneidlinien nach innen gefaltet wird;
- Fig. 6: eine Draufsicht zur Erläuterung der Weise zeigt, in der der Teil des Lagenelementes, der nach innen gefaltet wurde, anschließend teilweise nach außen gefaltet wird; und
- Fig. 7: eine Draufsicht zur Erläuterung der Weise zeigt, in der zwei derartige Lagenelemente einander gegenüberliegend angeordnet werden, so daß jeweils zwei einander zugeordnete Ausschnitte die Öffnung bilden können.

### BEVORZUGTE AUSFÜHRUNGSFORMEN DER ERFINDUNG

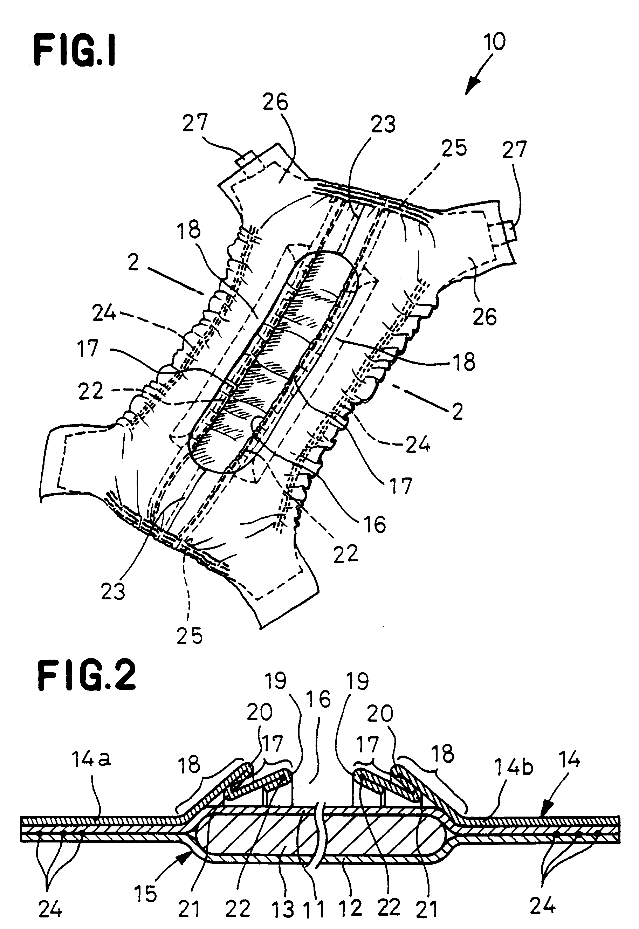

Wie in Fig. 1 und 2 gezeigt, umfaßt eine Windel 10 eine flüssigkeitsdurchlässige erste Decklage 11, eine flüssigkeitsundurchlässige Außenlage 12, einen flüssigkeitsabsorbierenden Kern 13, der zwischen diese gelegt ist, und eine flüssigkeitsbeständige zweite Decklage 14. Die zweite Decklage 14 ist in ihrem Mittelbereich mit einer Öffnung 16 versehen, die in Längsrichtung größer ist als in Querrichtung der Lage 14 und deren in Längsrichtung gegenüberliegende Enden jeweils Kreisbögen beschreiben. Die Öffnung 16 kann wenigstens innerhalb des Schrittbereiches ausgebildet sein.

Die zweite Decklage 14 umfaßt eigentlich zwei Lagenelemente 14a, 14b. Die Öffnung 16 ist an seitlich einander gegenüberliegenden Rändern mit ersten und zweiten Klappen 17, 18 versehen, die durch Falten der Innenabschnitte der jeweiligen Lagenelemente 14a, 14b gebildet werden. Genauer können die inneren Abschnitte der jeweiligen Lagenelemente 14a, 14b nach unten gefaltet werden, um die zweiten Klappen 18 zu bilden, die jeweils zweite innere Seitenränder 20 aufweisen, worauf die Abschnitte der jeweiligen Lagenelemente 14a, 14b, die sich von den jeweiligen zweiten Klappen nach innen erstrecken nach oben gefaltet werden können, worauf die innersten Abschnitte der jeweiligen Lagenelemente 14a, 14b nach unten gefaltet werden können, um die ersten Klappen 17 zu bilden, die jeweils einen ersten inneren Seitenrand 19 aufweisen. Es versteht sich, daß während der Bildung der jeweiligen Klappen die gegenüberliegenden Innenflächen der jeweiligen gefalteten Abschnitte unter Verwendung eines geeigneten Verbindungsmittels (z.B. Heißschmelzkleber oder Verschweißen) miteinander verbunden werden. Wie aus einem derartigen Prozeßablauf offensichtlich ist, sind die zweiten Klappen 18 kontinuierlich an die unteren Seitenränder 21 der jeweilgen ersten Klappen 17 anschließend ausgebildet, die zweiten Seitenränder 20 oberhalb der zugehörigen ersten Klappen 17 zwischen den ersten Seitenrändern 19 und den unteren Seitenrändern 21 derselben angeordnet und einander gegenüberliegende zweite innere Seitenränder 20 sind voneinander durch eine Strecke beabstandet, die größer ist als die Strecke, durch die die einander gegenüberliegend angeordneten ersten inneren Seitenränder 19 voneinander beabstandet sind. In den ersten inneren Seitenrändern 19 sind jeweils in Längsrichtung dehnbare elastische Elemente 22 enthalten, die jeweils einen einzelnen oder mehrere elastische Fäden umfassen, die in den Seitenrändern unter Verwendung von Heißschmelzkleber (nicht dargestellt) in gedehntem Zustand angebracht sind. Die zweiten inneren Seitenränder 20 können ebenfalls ähnliche elastische Elemente jeweils enthalten, falls dies erwünscht ist.

Wie in Fig. 1 gezeigt, sind die einander gegenüberliegend angeordneten inneren Seitenränder 23 der Lagenelemente 14a, 14b, die einander in der Nähe der in Längsrichtung gegenüberliegenden Enden überlappen, in der Art und Weise miteinander verbunden, daß die Hilfsperson zum Windelanlegen sie ohne weiteres voneinander trennen kann. Genauer können diese einander gegenüberliegend angeordneten inneren Seitenränder 23 entweder unter Verwendung von Heißschmelzkleber miteinander verklebt werden oder punktverschweißt werden.

Zwischen der ersten Decklage 11 und der Außenlage 12, die sich beide über die seitlich gegenüberliegend angeordneten Seiten des flüssigkeitsabsorbierenden Kernes 13 nach außen erstrecken, sind nahe an den äußeren Rändern dieser Lagen mehrere parallel verlaufende elastische Elemente 24 vorgesehen, die jeweils wiederum mehrere elastische Fäden umfassen, die in gedehntem Zustand unter Verwendung von Heißschmelzkleber (nicht dargestellt) jeweils befestigt sind, so daß sie in Längsrichtung der Lagen dehnbar sind und somit dicht um die Beine des Trägers anliegen. In ähnlicher Weise sind zwischen den in Längsrichtung gegenüberliegenden Enden der ersten Decklage 11 und den zugehörigen Enden der Außenlage 12 mehrere elastische Elemente 25 vorgesehen, die jeweils mehrere elastische Fäden umfassen, so daß sie quer zu diesen Lagen dehnbar sind und damit dicht um die Hüfte des Trägers anliegen.

Die erste Decklage 11 kann aus Vliesstoff, poröser Kunststoffolie oder ähnlichem, die Außenlage 12 aus Kunststoffolie, einer geschichteten Lage dieser Kunststoffolie und Vliesstoff oder ähnlichem, und der flüssigkeitsabsorbierende Kern 13 aus einer Mischung von Faserpulpe und hoch absorbierendem Polymerpulver oder ähnlichem hergestellt sein. Die zweite Decklage 14 ist vorzugsweise aus wasserabstoßendem und hoch luftdurchlässigem Vliesstoff hergestellt. Es versteht sich, daß in der Beschreibung der Erfindung sich der Begriff "flüssigkeitsbeständiges" Material auf Material bezieht, das einen ausreichenden Grad von wasserabstoßenden Eigenschaften aufweist, um zu verhindern, daß flüssige Ausscheidungen leicht dieses durchdringen, wenn die Windel am Körper des Trägers getragen wird.

Wie wiederum Fig. 1 zeigt, weist die Windel 10 zwei flügelähnliche Klappen 26 auf, die sich von den seitlich einander gegenüberliegenden Seiten der Hüftlinie jeweils nach außen erstrecken, und die freien Enden von Befestigungsbändern 27, die an der Rückseite der flügelähnlichen Klappen angebracht sind, können an der Außenlage 12 an der Vorderseite durch Kleben befestigt werden, um die Windel 10 um den Körper des Trägers aufzurichten.

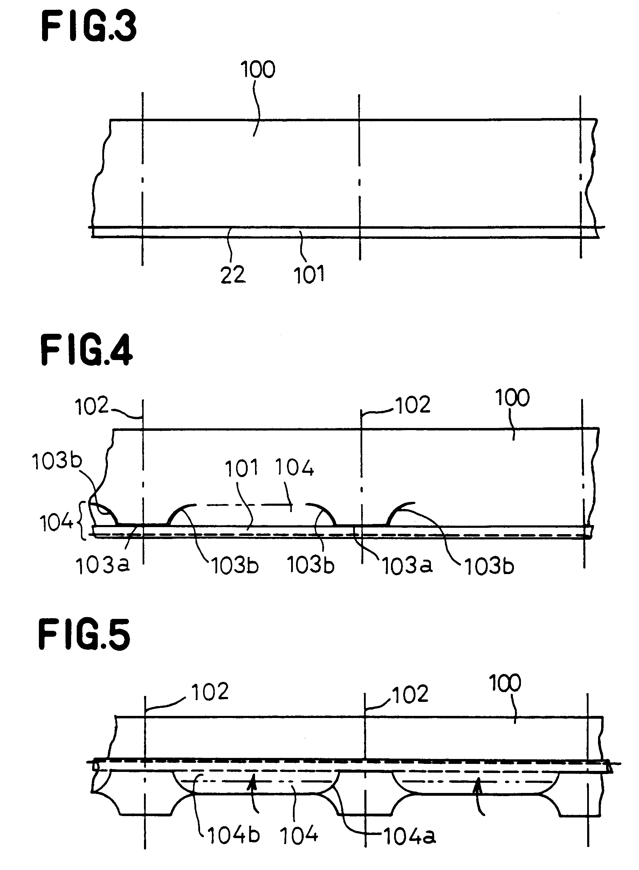

Nachfolgend werden die Vorgänge zur Bildung der Öffnung 16, der ersten und zweiten Klappen 17, 18 und zum Anbringen der elastischen Elemente 22 unter Bezug von Fig. 3 bis 7 erläutert.

Wie Fig. 3 zeigt, werden endlose elastische Elemente 22 unter Dehnung mit einem vorgegebenen Prozentsatz an einem endlosen Lagenelement 100 (d.h. einer endlosen Bahn, die die Lagenelemente 14a, 14b umfaßt) nahe entlang einem Seitenrand, der sich in Längsrichtung der Lage erstreckt, unter Verwendung von Heißschmelzkleber (nicht dargestellt) angebracht.

Wie aus Fig. 4 ersichtlich ist, wird ein Randbereich 101, der die an der Lage angebrachten elastischen Elemente 22 einschließt, umgefaltet und mit der gegenüberliegenden Oberfläche unter Verwendung von Heißschmelzkleber oder Schweißung verbunden. Anschließend wird das endlose Lagenelement 100 mit geraden Schnittlinien 103a versehen, die sich entlang den inneren Seitenrändern des Randbereiches 101 über jeweilige Linien 102 erstrecken, die sich wiederum quer zum endlosen Lagenelement 100 erstrecken und entlang welchen das endlose Lagenelement 100 später geschnitten wird, um die einzelnen Windeln zu bilden. Zusätzlich wird das endlose Lagenelement 100 mit kreisbogenförmigen Schnittlinien 103b versehen, die von den Enden der jeweiligen geraden Schnittlinien 103a ausgehen.

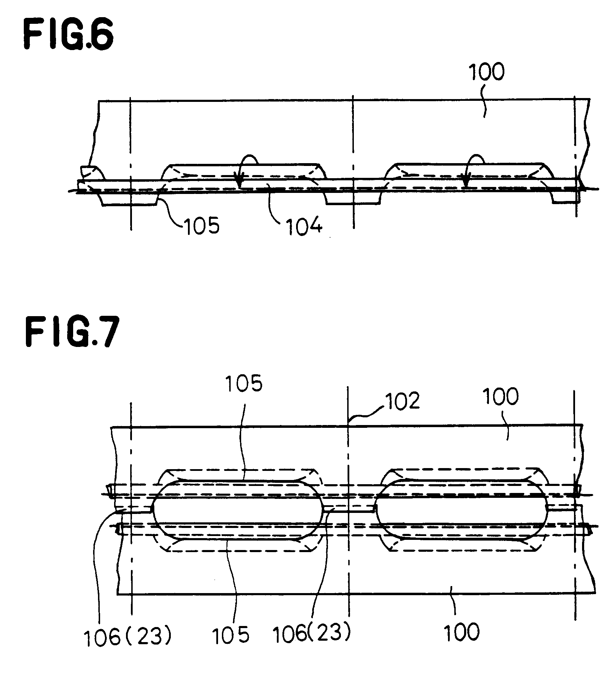

Wie Fig. 5 zeigt, wird ein jeweiliger Bereich 104 (der in die ersten und zweiten Klappen 17, 18 umzubilden ist), der von der geraden und den kreisbogenförmigen Schnittlinien 103a, 103b einschließlich des Randbereiches 101 begrenzt wird, entlang einer geraden Linie, die die inneren Enden der kreisbogenförmigen Schnittlinien 103b zwischen jeweils zwei benachbarten Linien 102 verbindet, nach innen gefaltet.

In Fig. 5 und 6 bezeichnet Bezugszeichen 104a einen Abschnitt, der ein Teil des Bereiches 104 ist, und Bezugszeichen 104b bezeichnet einen Abschnitt (der zur ersten Klappe 17 umzubilden ist), dessen Breite annähernd eine Hälfte des Abschnittes 104a beträgt, wobei ersterer Abschnitt nach außen gefaltet wird.

Wie Fig. 7 zeigt, werden zwei der Lagenelemente 100, die jeweils die Öffnungen 16 bildende Ausschnitte 105 sowie die ersten Klappen 17, in denen elastische Elemente 22 enthalten sind, und die zweiten Klappen 18 umfassen, auf die obere Fläche einer endlosen Windelbahn (nicht dargestellt), die die einzelnen Windeln, wie in Fig. 1 und 2 dargestellt, umfaßt (mit der Ausnahme, daß die zweite Decklage 14 fehlt), als Schicht aufgelegt, so daß jeweils zwei einander beiderseits gegenüberliegend angeordneten Ausschnitte 105 miteinander fluchtend ausgerichtet werden, um die Öffnung 16 zu bilden, und jeweils zwei beiderseits einander gegenüberliegend angeordnete innere Seitenränder 106 (die den inneren Seitenrändern 23 entsprechen) einander überlappen, worauf sie unter Verwendung von Heißschmelzkleber oder ähnlichem an den gewünschten Stellen mit der Windelbahn verbunden werden und diese dergestalt geschichtete Bahn an den Linien 102 geschnitten wird, um die einzelnen Windeln, wie in Fig. 1 und 2 dargestellt, zu erhalten.

Während die Ausführungsform als sogenannte Windel des offenen Typs erläutert und beschrieben wurde, die zum Schließen der Hüftlinie um den Körper des Trägers die Befestigungsbänder verwendet, ist die Erfindung auch auf sogenannte Windeln des Höschentyps (einschließlich Erziehungshöschen) mit einer endlosen Hüftlinie anwendbar.

Die erfindungsgemäße Windel, die wie vorstehend beschrieben angeordnet wird und funktioniert, verringert auf wirksame Weise die Gefahr, daß die Haut des Trägers mit einem Teil von Ausscheidungen verschmiert wird, der die Öffnung, die in der zweiten Decklage, die direkt die Haut des Trägers berührt, ausgebildet ist, verfehlt haben, was dem Träger unangenehme Gefühle vermittelt und sogar die Ursache dafür sein kann, daß der Träger Hautkrankheiten erleidet. Dieser Teil der Ausscheidungen wird somit zuverlässig von den zweiten Klappen eingedämmt und dadurch daran gehindert, sich über die obere Fläche der zweiten Decklage zu verteilen.

Gemäß einem zusätzlichen Merkmal der Erfindung umfaßt die zweite Decklage zwei Lagenelemente, deren innere Seitenränder miteinander in der Nähe der in Längsrichtung gegenüberliegend vorliegenden Enden derselben miteinander verbunden sind, so daß diese verbundenen inneren Seitenränder getrennt werden können, um die erste Decklage, die unter der zweiten Decklage liegt, freizulegen, so daß auf der ersten Decklage befindliche feste Ausscheidungen leicht abgekratzt werden können.

## Patentansprüche

1. Wegwerfwindel (10), umfassend eine flüssigkeitsdurchlässige erste Decklage (11), eine flüssigkeitsundurchlässige Außenlage (12), einen flüssigkeitsabsorbierenden Kern (13), der zwischen die erste Decklage (11) und die Außenlage (12) gelegt ist, und eine flüssigkeitsbeständige zweite Decklage (14), die über der ersten Decklage (11) liegt, wobei die zweite Decklage (14) in wesentlichen in ihrem Mittelbereich mit einer Öffnung (16) versehen ist, die sich in Längsrichtung weiter als in Querrichtung der zweiten Decklage (14) erstreckt, wobei die zweite Decklage (14) entlang ihrem äußeren Umfang mit der ersten Decklage (11) verbunden ist, und wobei in Längsrichtung elastische Elemente (22) an der zweiten Decklage (14) entlang dem Umfangsrand der Öffnung (16) angebracht sind, **dadurch gekennzeichnet, daß**
die Öffnung (16) mit zwei ersten Klappen (17) versehen ist, die jeweils erste innere Seitenränder (19) aufweisen, die einander gegenüberliegend und voneinander beabstandet angeordnet sind,
und zwei zweite Klappen (18), die jeweils zweite innere Seitenränder (20) aufweisen, die einander gegenüberliegend und zueinander durch eine Strecke beabstandet sind, die größer ist als die Strecke, durch die die ersten inneren Seitenränder (19) voneinander beabstandet sind,
wobei die beiden zweiten Klappen (18) sich kontinuierlich an die unteren Seitenränder (21) der jeweiligen ersten (17) Klappen anschließen,
und die zweiten inneren Seitenränder (20) zwischen den ersten inneren Seitenrändern (19) und den zugehörigen unteren Seitenrändern (21) der jeweiligen ersten (17) Klappen positioniert sind, so daß diese zweiten inneren Seitenränder (20) sich nach oben über die oberen Flächen der jeweiligen ersten Klappen (17) erheben können
und wenigstens in den ersten inneren Seitenrändern (19) jeweils elastische Elemente (22) enthalten sind.

2. Windel nach Anspruch 1, wobei die ersten und zweiten Klappen (17,18) durch Falten der einander gegenüberliegend angeordneten inneren Ränder der Öffnung (16) gebildet werden.

3. Windel nach Anspruch 1, wobei die zweite Decklage (14) zwei Lagenelemente (14a,14b) umfaßt, die jeweils die ersten und zweiten Klappen (17,18) einschließen.

4. Windel nach Anspruch 3, wobei die inneren Seitenränder (23) der Lagenelemente (14a,14b), die einander beiderseits gegenüberliegend in der Nähe der in Längsrichtung gegenüberliegenden Enden derselben vorliegen, miteinander verbunden werden, so daß diese verbundenen inneren Seitenränder (23) voneinander getrennt werden können, ohne daß in den Lagenelementen (14a,14b) selbst Risse auftreten.

## Claims

1. A disposable nappy (10), comprising a first cover sheet (11) permeable to liquids, an outer sheet (12) impermeable to liquids, a liquid-absorbing core (13) which is disposed between the first cover sheet (11) and the outer sheet (12), and a second cover sheet (14) resistant to liquids which lies over the first cover sheet (11), wherein the second cover sheet (14) is provided with an opening (16) substantially in its middle region which extends further in the longitudinal direction than in the transverse direction of the second cover sheet (14), wherein the second cover sheet (14) is connected to the first cover sheet (11) along its outer periphery and wherein elastic elements are applied to the second cover sheet (14) in the longitudinal direction along the peripheral edge of the opening (16), characterized in that
the opening (16) is provided with two first flaps (17) which have respective first inner side edges (19) which are arranged opposite and spaced from each other,
and second flaps (18) which have respective second inner side edges (20) which are spaced opposite one another by a distance which is greater than the distance by which the first inner side edges (19) are spaced from one another,
wherein the two second flaps (18) continuously join on to the underneath side edges (21) of the respective first flaps (17),
and the second inner side edges (20) are located between the first inner side edges (19) and the associated underneath inner side edges (21) of the respective first flaps (17), so that these second inner side edges (20) can lift up above the upper surfaces of the respective first flaps (17),
and respective elastic elements (22) are included at least in the first inner side edges (19).

2. A nappy according to claim 1, wherein the first and second flaps (17, 18) are formed by folding the mutually opposed inner edges of the opening (16).

3. A nappy according to claim 1, wherein the second cover layer (14) comprises two layer elements (14a, 14b) which include the first and second flaps (17, 18).

4. A nappy according to claim 3, wherein the inner side edges (23) of the layer elements (14a, 14b) which lie opposite one another on both sides in the vicinity of the ends thereof in the longitudinal direction are joined together, so that these joined inner side edges (23) can be separated from one another, without tears occurring in the layer elements (14a, 14b) themselves.

## Revendications

1. Lange jetable (10), comportant une première couche de recouvrement (11) perméable aux liquides, une couche extérieure (12) imperméable aux liquides, un corps central absorbant (13), emprisonné entre la première couche de recouvrement (11) et la couche extérieure (12), et une seconde couche de couverture (14) à l'épreuve des liquides, posée sur la première couche de recouvrement (11), ladite seconde couche de couverture (14) étant principalement pourvue, dans sa zone centrale, d'une ouverture (16) de dimension plus importante dans le sens longitudinal de la seconde couche de couverture (14) que dans le sens transversal de cette dernière, lange dans lequel la seconde couche de couverture (14) est jointe à la première (11) sur son pourtour extérieur, et dans lequel des éléments élastiques (22) sont incorporés dans le sens longitudinal à la seconde couche de couverture (14), sur le pourtour du bord périphérique de l'ouverture (16), ledit lange étant caractérisé en ce que :
l'ouverture (16) est dotée de deux premiers rabats (17) présentant, chacun, un premier bord latéral interne (19), ces premiers bords latéraux internes étant mutuellement opposés et espacés,
et deux seconds rabats (18) présentant, chacun, un second bord latéral interne (20), ces seconds bords latéraux internes étant mutuellement opposés et espacés d'une distance plus grande que la distance séparant les deux premiers bords latéraux internes (19),
les deux seconds rabats (18) étant joints, sans discontinuité, aux bords latéraux inférieurs (21) de chaque premier rabat (17),
et les seconds bords latéraux internes (20) se situant entre les premiers bords latéraux internes (19) et les bords latéraux inférieurs associés (21) de chaque premier rabat (17), de sorte que ces seconds bords latéraux internes (20) puissent se soulever (au-dessus) des faces supérieures de chaque premier rabat (17),
et des éléments élastiques (22) étant incorporés au moins à chacun des premiers bords latéraux internes (19).

2. Lange selon la revendication 1, dans lequel les premiers et seconds rabats (17, 18) sont formés en repliant les bords intérieurs de l'ouverture (16) mutuellement opposés.

3. Lange selon la revendication 1, dans lequel la seconde couche de couverture (14) comprend deux parties de couche (14a, 14b), lesquelles comportent, chacune, les premiers et seconds rabats (17, 18).

4. Lange selon la revendication 3, dans lequel les bords latéraux internes (23) des parties de couche (14a, 14b) qui se trouvent en opposition, de chaque côté, au voisinage des extrémités longitudinales opposées desdites parties de couche, sont raccordés entre eux, en sorte que ces bords latéraux internes (23) reliés puissent être séparés l'un de l'autre, sans que des déchirures se produisent dans les parties de couche (14a, 14b) proprement dites.
